# EUROPEAN PATENT APPLICATION

(11) **EP 3 192 449 A1**
(43) Date of publication of application: **19.07.2017**
(21) Application number: 16207010.6
(22) Date of filing: 27.12.2016
(51) Int. Cl.: A61B 8/12, A61B 8/00

(54) **SYSTEM FOR ROBOT-ASSISTED CONTROL OF A TRANSRECTAL PROBE, FOR EXAMPLE FOR USE IN CARRYING OUT PROSTRATE ECHOGRAPHY**

(30) Priority: 14.01.2016 IT UB20169976
(71) Applicant: Comau S.p.A., 10095 Grugliasco (Torino) (IT)
(72) Inventor: Ippolito, Mr. Massimo, I-10095 Grugliasco (Torino) (IT); Bordegnoni, Mr. Stefano, I-10095 Grugliasco (Torino) (IT)
(74) Representative: Notaro, Giancarlo

(57) **Abstract**

A system (1) for controlling a transrectal probe, for example a transrectal ultrasound probe for echography, comprises a probe (2) and a structure (3) for supporting the probe (2) that is carried by the wrist (6) of a multi-axis manipulator robot (4). Operatively set between the wrist (6) of the robot (4) and the probe (2) is a load cell (5). The system further comprises an electronic control unit (E) for controlling the robot (4) and a manual-guide device (7, 8) for guiding the robot, which is connected to the electronic control unit (E) of the robot (4) and comprise a manual-guide member (71, 83), which can be manoeuvred by an operator for imparting on the robot (4) movements that are function of the movements imparted on the manual-guide member (71, 83). The electronic control unit (E) of the robot (4) receives signals emitted by the load cell (5) and activates an alarm condition when the load cell (5) signals that a threshold value of the stress detected thereby is exceeded. Preferably, the manual-guide member (71, 83) is pre-arranged for supplying to the operator a haptic feedback, for example of a vibrational type.

## Description

### TEXT OF THE DESCRIPTION

### Field of the invention

The present invention relates in general to the field of systems and devices for control of transrectal probes and regards in particular control of a transrectal ultrasound probe for echography, for example of the type used during execution of a prostate biopsy.

### Prior art

Already in use are devices of the type indicated above comprising an ultrasound probe carried by a supporting structure connected to the distal end of an articulated arm. These devices assist the operator in maintaining precisely a desired position of the ultrasound probe, but are in any case not very easy to use, also due to the weight of the structure that must be supported by the operator.

Likewise already known are attempts at providing transrectal probes controlled by robots. For example, the patent document No. US 2015/0265354 filed in the name of John Hopkins University describes a robot-controlled transrectal probe of an MRI (Magnetic Resonance Imaging) type. None of these attempts has, however, proven altogether satisfactory owing to the difficulties of guaranteeing safety for the patient and at the same time convenient and precise control by the operator.

### Object of the invention

The object of the present invention is to overcome the drawbacks of the known art.

More in general, the object of the invention is to provide a system for robot-assisted control of a transrectal probe of any type that will be extremely convenient and easy to use for the physician, will guarantee the necessary safety for the patient, will enable an extremely precise control of movement and positioning of the probe, and finally will present a relatively contained cost of production and maintenance.

### Summary of the invention

With a view to achieving the above purposes, the subject of the invention is a system for controlling a transrectal probe, for example a transrectal ultrasound probe for echography, comprising:
- a transrectal probe;
- a structure for supporting said probe;
- a multi-axis manipulator robot having a wrist that carries said structure for supporting said probe;
- a load cell operatively set between said robot wrist and said transrectal probe;
- an electronic control unit for controlling said robot;
- a manual-guide device for guiding said multi-axis manipulator robot, which is connected to said electronic control unit of the robot and comprises a manual-guide member, which can be manoeuvred by an operator for imparting on the robot movements that are a function of the movements imparted on said guide member,
- said electronic control unit of the robot being programmed for controlling said robot on the basis of the movement manually imparted by an operator on said manual-guide member, in view of driving the probe inside the rectum of a patient;
- said electronic control unit of the robot being moreover programmed for receiving signals emitted by said load cell and activating an alarm condition when said load cell signals that a threshold value of the stress detected thereby is exceeded.

Thanks to the aforesaid characteristics, the system according to the invention enables the operator to manoeuvre the probe in an extremely easy and convenient way, although ensuring an extremely precise control of the movement and positioning of the probe, and maximum safety for the patient.

The aforesaid alarm condition activated by the electronic control unit of the robot may consist in arrest of the robot and/or in emission of a visual alarm signal and/or an acoustic alarm signal.

In a preferred embodiment, the aforesaid manual-guide member is pre-arranged for supplying to the operator a haptic feedback, for example of a vibrational type. Preferably, the haptic feedback has an intensity proportional to the stress detected by said load cell.

Once again in the case of the preferred embodiment, the manual-guide device is rigidly connected, either directly or indirectly, preferably in a removable way, to the robot, and in particular to an element that is mobile together with the distal end of the robot.

### Detailed description of preferred embodiments

Further characteristics and advantages of the invention will emerge from the ensuing description with reference to the annexed drawings, which are provided purely by way of non-limiting example and in which:
- Figure 1 is a schematic view of the system according to the invention;
- Figure 1A is a view at an enlarged scale of some details illustrated in Figure 1;
- Figure 2 is a block diagram that illustrates operation of the system of Figure 1; and
- Figures 3 and 4 show two perspective views of a manual-guide device illustrated in Figure 1.

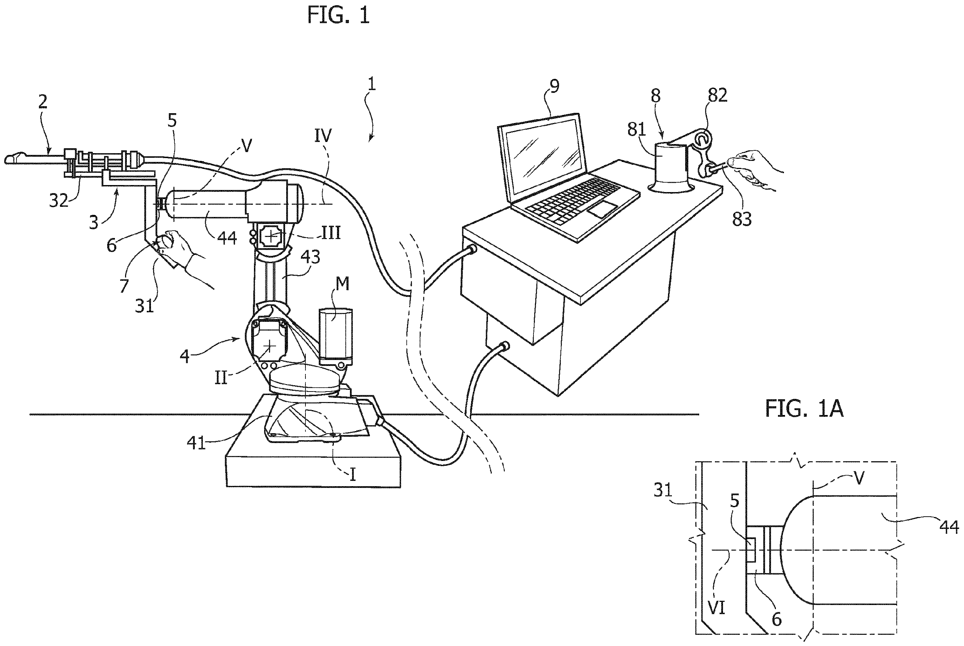

Figure 1 illustrates a system 1 according to the present invention, for robot-assisted control of a transrectal probe. The example illustrated herein regards the case of an ultrasound probe that can be used, for example, for carrying out a prostate biopsy. Any ultrasound probe of a known type may be suitable for the purpose. For example, the present applicant has conducted first experiments with an ultrasound probe produced by the company BK Ultrasound Systems and marketed under the tradename BioJet Fusion. It should in any case be noted that the present invention is of general application and is suited to being used for controlling a transrectal probe of any type.

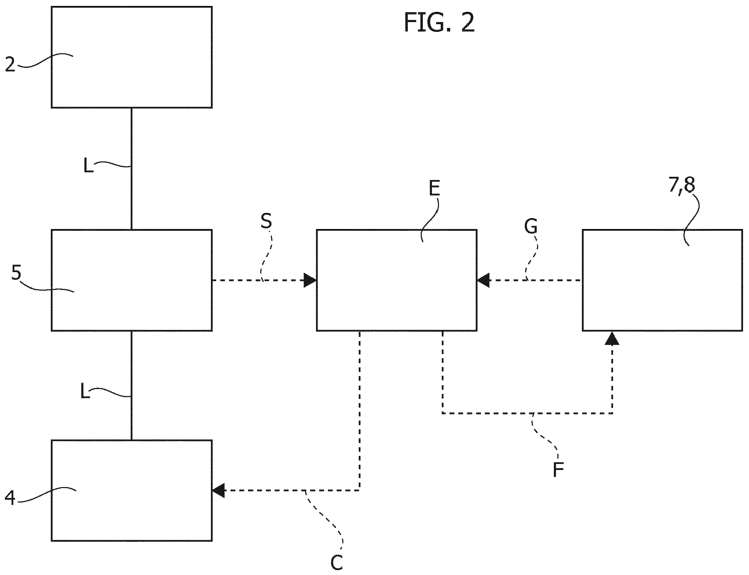

The system 1 comprises a multi-axis manipulator robot 4. In the example illustrated, the robot 4 is an anthropomorphic robot having a base 41 and a column 42, which is rotatably mounted on the base 41 about a first axis I directed vertically. The robot 4 has an arm 43 mounted on the column 42 articulated about a second axis II directed horizontally. Designated by the reference 44 is a forearm mounted on the arm 43. The forearm 44 is articulated about a third axis III, which is also directed horizontally; the forearm 44 moreover has the possibility of rotating about its longitudinal axis IV. The forearm 44 of the robot 4 is provided at its end opposite to the arm 43 with a wrist 6 mounted with possibility of rotation about two mutually orthogonal axes V, VI. According to the known art, each of the six axes I, II, III, IV, V, and VI of the robot is controlled by a respective electric motor (in the drawing only the electric motor M that controls the first axis I is visible) via a respective reducer. The electric motors of the robot are controlled in a way in itself known by an electronic control unit E (illustrated in the block diagram of Figure 2). In an example of embodiment that has formed the subject of the studies and experiments conducted by the present applicant, the robot produced by the present applicant under the tradename "RACER 3" has proven particularly suited.

In accordance with the known art, provided at the distal end of the robot wrist 6 is a flange for attachment of the tool carried by the robot. In the case of the present invention, instead of a tool, associated to the flange of the robot 4 is a supporting structure 3 carrying a transrectal ultrasound probe 2. In the embodiment illustrated in Figure 1, the supporting structure 3 is constituted by a substantially L-shaped main body 31, associated to which is a supporting frame 32 connected on which is the transrectal probe 2. It is, however, to be noted that the structure 3 for supporting the probe 2 may have any configuration different from the one illustrated herein purely by way of example.

According to an essential characteristic of the present invention, the system 1 further comprises a load cell 5 operatively set between the wrist 6 of the robot 4 and the transrectal probe 2. Figure 1A is a schematic view at an enlarged scale of the terminal end of the robot 4, which shows in particular the load cell 5 set between the flange at the distal end of the robot 4 and the main body 31 of the structure 3 that supports the probe 2. More in general, it is necessary for the load cell 5 to be interposed in any point of the chain of elements that mechanically connect the probe 2 to the distal end of the robot 4.

The load cell 5 is used in the system 1 in order to detect, preferably in a continuous way, the load exchanged between the transrectal probe 2 and the wall of the patient's rectum. In the present description, and in the ensuing claims, by the expression "load cell" is meant any type of sensor device designed to detect a force or a load. This expression encompasses the case of a unidirectional sensor, designed to detect forces acting only in one direction (for example, the longitudinal direction of the probe 2) and the case of a sensor, or system of sensors, designed to detect forces and/or couples acting in a number of directions.

In a simplified example of embodiment, the load cell 5 is provided with one or more electrical strain gauges. The mechanical stress on the probe 2 is measured in a way in itself known, via detection of a variation of electrical resistance in the electrical circuit of the strain gauges.

As already mentioned, the transrectal ultrasoundprobe for echography may be of any known type available on the market. The probe is available in association with a processing and display device and a corresponding processing program. The processing and display device may, for example, be in the form of a portable personal computer, such as the one designated by 9 in Figure 1.

According to a further important characteristic of the invention, the system 1 comprises a manual-guide device for guiding the multi-axis manipulator robot 4, which is connected to the electronic control unit E of the robot (see also the block diagram of Figure 2) and comprises a manual-guide member, which can be manoeuvred by the physician for imparting on the robot 4 movements that are a function of the movements imparted on the manual-guide member.

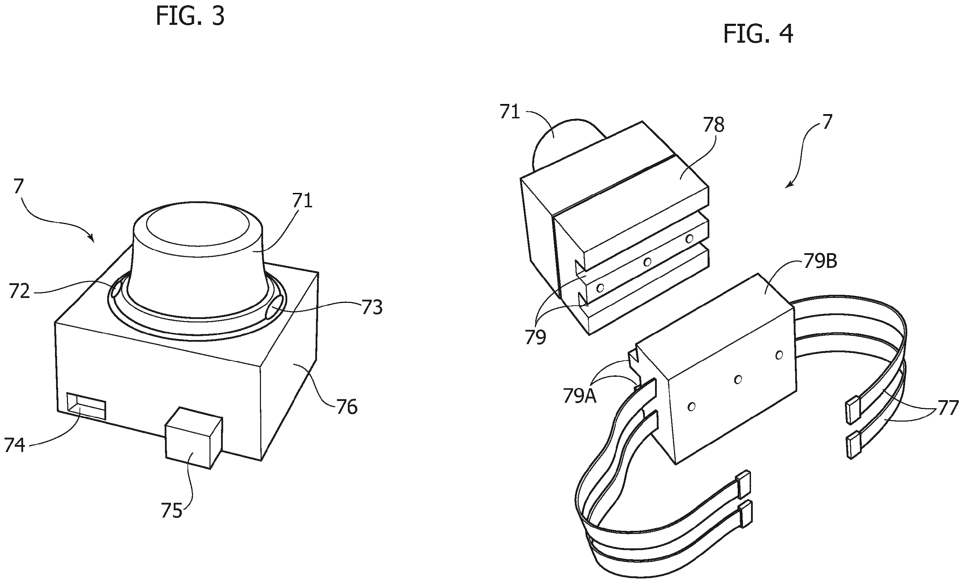

Once again with reference to Figure 1, illustrated simultaneously therein are two different manual-guide devices 7 and 8 of the robot, which can be used as an alternative to one another. The preferred embodiment is the one that makes use of a manual-guide device 7 associated to the distal end of the robot. Figures 3 and 4 illustrate two perspective views of the manual-guide device 7 that is of the known type illustrated in the document EP 2 194 434 A1 filed in the name of the present applicant. In the example illustrated in Figure 1, the manual-guide device 7 is associated to the supporting structure 3 of the probe 2. According to what is already known from the document EP 2 194 434 A1, the guide device 7 has inside it a microprocessor electronic control system of a programmable type, and permanent rewriteable memory means. Designated by the reference 71 is the manual-guide member of the device 7, which is constituted by a knob with a number of degrees of freedom. The device 7 further comprises some pushbuttons, amongst which a pushbutton 72 for switching on and switching off the device 7 and a storage pushbutton 73 that can be used by the operator for storing coordinates of path of the manipulator robot 4. The device 7 is also conveniently provided with a connector 74 for connection to a charging device of a known type (not represented herein).

Once again in the case of the preferred embodiment, the manual-guide device 7 is provided with wireless-communication means in order to establish a wireless-communication channel with the electronic control unit of the robot E. For this purpose, designated by the reference 75 is a wireless transceiver module, which is connected to the electronic control unit E.

The device 7 illustrated in Figures 3 and 4 further comprises a casing of a prismatic shape 76 present within which is the corresponding control system and carried on which is the knob 71. The control circuit of the device 7 is of a miniaturized type so that the dimensions of the casing 76 are rather small.

The device 7 is moreover provided with a plurality of straps 77 for quick fixing to the supporting structure 3. Of course, instead of the straps 77 there may be used other mounting means suitable for use on the supporting structure 3.

In the example of Figure 4, the casing 76 has a respective base 78 that defines one or more engagement seats 79, which are designed to receive respective portions 79a of the mounting base 79b on which the straps 77 are applied.

The knob 71 preferably has six degrees of freedom. For example, by exerting a pressure or else a pull in the axial direction on the knob 71 there is brought about advance or recession of the forearm 44 of the manipulator robot 4. By pressing the knob to the right or to the left there is obtained, respectively, a displacement to the right and to the left of the forearm 44. Likewise, by pushing the knob downwards (i.e., towards the south of the knob) or upwards (i.e., towards the north of the knob) there are obtained the corresponding movements of the forearm 44. The knob 71 may moreover be rotated in a clockwise and counterclockwise direction to obtain corresponding relative movements of rotation.

As indicated previously, in Figure 1 designated by the reference 8 is a second type of manual-guide device alternative to the manual-guide device 7. The manual-guide device 8 is of the 6-degrees-of-freedom mouse type and is constituted by a base 81 of a cylindrical shape which can be constrained to a plane surface (in the example of Figure 1, the base 81 is constrained to a table on which the computer 9 rests). Connected to the base 81 is a mobile arm 82 connected to the terminal end of which is the guide member 83, which, in the embodiment illustrated in Figure 1, is a lever control device of a joystick type. Like the device 7 described previously, also the device 8 has relatively contained dimensions. A device of this type that is currently available on the market is the device BioJet Fusion manufactured by the company BK Ultrasound Systems. For this reason, the constructional details of this device are not illustrated any further herein.

Figure 2 illustrates a functional diagram of the system 1 according to the present invention. The lines L indicate from the functional standpoint the fact that the load cell 5 is set between the flange at the distal end of the robot 4 and the probe 2. The electronic control unit E of the robot 4 receives control signals G from the manual-guide device 7 or 8 following upon manual actuation of the corresponding guide member by the physician. The electronic unit E sends control signals C to the robot 4 on the basis of the control signals G coming from the manual-guide device 7 or 8. In this way, the physician can control movement of the probe in an easy and intuitive way and without having to make any effort. In the case of an ultrasound probe, the operator drives the probe via the manual-guide device, controlling in real time the image of the organ (for example, the prostate) obtained via the probe.

According to a further characteristic of the invention (see once again Figure 2), the electronic control unit E is programmed for receiving the signals S emitted by the load cell 5 and for activating an alarm condition in the case where the stress detected by the load cell 5 exceeds a predetermined threshold value. The alarm condition activated by the electronic unit E may consist in arrest of the robot 4 and/or in activation of a visual and/or acoustic alarm, for example emitted by an emitter pre-arranged on the manual-guide device 7 or 8. Thanks to the above characteristic, the system according to the invention guarantees the necessary safety for the patient, notwithstanding the fact that the operator moves the transrectal probe via the robot 4.

According to a further characteristic of the preferred embodiment of the system according to the invention, the manual-guide member (71 or 83) of the manual-guide device (7 or 8) is pre-arranged for supplying the operator with a haptic feedback, for example of a vibrational type, which is generated on the basis of a signal F produced by the electronic control unit E as a function of the signals S coming from the load cell 5.

In this way, use of the system according to the invention is even easier and more intuitive, in so far as the operator is provided with a direct feel of the degree of resistance that the probe encounters in its movement.

According to this preferred characteristic of the invention, the guide member 71, 83 consequently comprises inside it a haptic device of any known type so that the operator can receive tactile sensations in response to the displacements of the probe 2. Haptic devices have been known and used for some time now. These devices may, for example, make use of piezoelectric elements pre-arranged under the outer surface of a knob and designed to be set in vibration following upon an electrical signal sent to them.

The system may be pre-arranged for generating the haptic feedback only when the aforesaid threshold value of the stress detected by the load cell 5 is exceeded. However, preferably, the system is pre-arranged for generating a haptic feedback on the guide member 71, 83, such as a vibrational haptic feedback, which is of an intensity that increases proportionally to the stress detected by the load cell 5. In this case, the operator can receive an increasing haptic signal even before the alarm condition is reached.

Preferably, the electronic unit E is programmed for getting the robot 4 to carry out an automatic initial manoeuvre of approach of the probe 2 to its starting position, where then the operator assumes total control of the probe via the manual-guide device of the robot.

As is evident from the foregoing description, the system according to the invention enables a robot-assisted control of a transrectal probe of any type, which is extremely convenient and intuitive to use for the physician and at the same time guarantees the necessary safety for the patient. The system according to the invention moreover enables an extremely precise control of the movement and positioning of the probe and makes use of means that are relatively simple and inexpensive.

Of course, without prejudice to the principle of the invention, the structural details and the embodiments may vary widely with respect to what has been described and illustrated herein merely by way of example, without thereby departing from the scope of the present invention as specified in the ensuing claims.

## Claims

1. A system (1) for controlling a transrectal probe, for example a transrectal ultrasound probe for echography, comprising:
- a transrectal probe (2);
- a supporting structure (3) for supporting said probe (2);
- a multi-axis manipulator robot (4) having a wrist (6) that carries said supporting structure (3) of said probe (2);
- a load cell (5) operatively set between said robot wrist (6) and said transrectal probe (2);
- an electronic control unit (E) for controlling said robot (4);
- a manual-guide device (7, 8) for guiding said multi-axis manipulator robot (4), which is connected to said electronic control unit (E) of the robot (4) and comprises a manual-guide member (71, 83), which can be manoeuvred by an operator for imparting on the robot (4) movements that are function of the movements imparted on said manual-guide member (71, 83);
- said electronic control unit (E) of the robot (4) being programmed for controlling said robot (4) on the basis of the movement imparted manually by an operator on said manual-guide member (71, 83), in view of driving the probe (2) inside the rectum of a patient;
- said electronic control unit (E) of the robot (4) being moreover programmed for receiving signals emitted by said load cell (5) and activating a alarm condition when said load cell (5) signals that a threshold value of the stress detected thereby is exceeded.

2. The system (1) according to Claim 1, **characterized in that** said alarm condition activated by said electronic control unit (E) of the robot (4) causes arrest of the robot (4).

3. The system (1) according to Claim 1 or Claim 2, **characterized in that** said alarm condition activated by said electronic control unit (E) causes emission of a visual alarm signal and/or an acoustic alarm signal.

4. The system (1) according to Claim 1, **characterized in that** said manual-guide member (71, 83) is pre-arranged for supplying to the operator a haptic feedback, for example of a vibrational type.

5. The system (1) according to Claim 1 or Claim 4, **characterized in that** said manual-guide device (7, 8) is rigidly connected, directly or indirectly, to said wrist (6) of the robot (4).

6. The system (1) according to Claim 5, **characterized in that** said manual-guide device (7, 8) is provided with means(77) for its anchorage to the robot (4) or to said supporting structure (3) of the probe (2).

7. The system (1) according to Claim 4, **characterized in that** said manual-guide device (7, 8) is pre-arranged for supplying a haptic feedback of an intensity proportional to the stress detected by said load cell (5).

8. A method for controlling a system (1) according to any one of the preceding claims, wherein:
- said robot (4) is controlled on the basis of the movement imparted manually by an operator on said manual-guide member (71, 83); and
- an alarm condition is activated when said load cell (5) signals that a threshold value of the stress detected thereby is exceeded.

## Amended claims

### Amended claims in accordance with Rule 137(2) EPC.

1. System (1) for controlling a transrectal probe, for example, a transrectal ultrasound probe for echography, comprising:
- a transrectal probe (2),
- a supporting structure (3) of said probe (2),
- a multi-axis manipulator robot (4) having a wrist (6) that carries said supporting structure (3) of said probe (2), said wrist being connected to a base (41) of the robot by means of a chain of robot elements (43, 44) mutually articulated about a plurality of robot axes (I, II, III, IV, V, VI),
- a load cell (5) operatively set between said robot wrist (6) and said transrectal probe (2), and
- an electronic control unit (E) of said robot (4), configured for receiving signals emitted by said load cell,
**characterized in that**:
- said robot comprises a plurality of electric motors (M) for controlling the rotation of aforesaid robot elements (43, 44) around to the respective axes (I, II, III, IV, V, VI),
- a manually-driven electronic guiding device (7, 8) of the robot, connected to said electronic control unit (E) of the robot (4), comprising a guide member (71, 83), which can be manoeuvred by an operator to generate control signals for said electronic control unit (E) of the robot (4) in order to drive said electric motors (M) in such a way as to impart movements to the robot (4) as a function of the movements imparted to said guide organ (71, 83), in view of driving the probe (2) inside the rectum of a patient,
- said electronic control unit (E) of the robot (4) also being programmed for receiving signals emitted by said load cell (5) and activating an alarm condition when said load cell (5) signals that a threshold value of the stress detected thereby is exceeded.

2. System (1) according to Claim 1, **characterized in that** said electronic guiding device (7, 8) is rigidly connected, directly or indirectly, to said wrist (6) of the robot (4) or to said supporting structure (3) of the probe (2).

3. System (1) according to Claim 2, **characterized in that** said electronic guiding device (7, 8) is provided with means (77) for its removable anchorage to the wrist (6) of the robot (4) or to said supporting structure (3) of the probe (2).

4. System (1) according to Claim 1, **characterized in that** said alarm condition activated by said electronic control unit (E) of the robot (4) causes arrest of the robot (4).

5. System (1) according to Claims 1 or 4, **characterized in that** said alarm condition activated by said electronic control unit (E) causes emission of a visual alarm signal and/or an acoustic alarm signal.

6. System (1) according to claim 1, **characterized in that** said guide organ (71, 83) is designed for providing a haptic feedback to the operator, for example, a vibrational feedback.

7. System (1) according to Claim 4, **characterized in that** said guide member (71, 83) is designed for providing a haptic feedback of an intensity proportional to the stress detected by said load cell (5).
